# EUROPEAN PATENT APPLICATION

(11) **EP 1 354 870 A1**
(43) Date of publication of application: **22.10.2003**
(21) Application number: 01271351.7
(22) Date of filing: 14.12.2001
(51) Int. Cl.: C07C 233/23, C07C 215/26, A61K 31/133, A61P 3/00

(54) **DIHYDROCERAMIDE DESATURASE INHIBITORS.**

(30) Priority: 19.12.2000 ES 200003045
(71) Applicant: CONSEJO SUPERIOR DE INVESTIGACIONES CIENTIFICAS, 28006 Madrid (ES)
(72) Inventor: FABRIAS DOMINGO, Gemma, I.I.Q.A J. Pascual Vila,, C/ Jorge Girona Salgado, 18-2 (ES); LLEBARIA SOLDEVILA, Amadeo, C/ Jorge Girona Salgado, 18-2 (ES); TRIOLA GUILLEM, Gemma, C/ Jorge Girona Salgado, 18-2 (ES)
(74) Representative: Carpintero Lopez, Francisco
(86) International application number: ES0100486
(87) International publication number: WO02050018

(57) **Abstract**

Cyclopropenylceramide derivatives as inhibitors of desaturases characterized by the general formula I in which R1 can be an alkyl, alkenyl, alkynyl, aryl or any heterocyclic group, n can have any value and it can be branched or straight chained and can contain unsaturated carbons and R2 and R3 can have the same or different values, and it can represent aryl, heteroaryl, alkyl or acyl groups with one or more unsaturated carbons on the chain, that can be branched or straight and substituted with OH groups. Members of this new class of compounds have shown an extraordinary activity as inhibitors of the dihydroceramide desaturase and of ceramide biosynthesis and are useful for the treatment of clinical conditions associated with increased intracellular ceramide.

## Description

### SECTOR OF THE TECHNIQUE

The present invention is of interest for the pharmaceutical industry. It refers to a technique for reducing intracellular ceramide levels that involves inhibition of dihydroceramide desaturase. The compounds described can be useful for the treatment of clinical conditions associated with a rise in intracellular ceramide both in humans and animals.

### STATE OF THE TECHNIQUE

Ceramide is an important lipidic effector (Hannun, J. Biol.. 269:3125 (1994), Merril, Jr., Nutr. Rev. 50:78 (1992), Kolesnick and Fuks, J. Exp. Med. 181: 1949 (1995), Chao, Mol. Cell. Neurosci. 6:91 (1995), Liscovitch, Trends Biochem. Sci. 17:393 (1992)). There are several extracellular and stress-inducing agents such as tumor necrosis factor α, interleukin-1 β, the 1-α-25-dihydroxyvitamin D₃, neurotrophins, the Fas ligand, dexamethasone, chemotherapeutic agents, ionizing radiation etc. that can induce a rise in endogenous ceramide levels (Hannun, J. Biol. Chem. 269:3125 (1994), Hannun and Obeid, Trends Biochem. Sci. 20:73 (1995), Ballou et al., J. Biol. Chem. 267:20044 (1992), Quintans et al., Biochem. Biophys. Res. Commun. 202: 710 (1994), Dobrowsky et al., Science 265: 1596 (1994), Yanaga and Watson, FEBS Lett. 314:297 (1992), Dressler and Kolesnick, Science 255:1715 (1992)). Intracellular ceramide acts as a mediator in response to external stimuli, in important processes such as cellular differentiation, apoptosis, suppression of cell growth etc. It has also been shown that exogenous ceramide analogues can provoke these same effects in different cell types (Hannun, J. Biol. Chem. 269:3125 (1994), Okazaki et al. J. Biol.. Chem. 265: 15823 (1990), Bielawska et al. FEBS Lett. 307:211 (1992), Obeid et al., Science 259: 1769 (1993), Laulederkind et al., J. Exp. Med. 182:599 (1995), Goldkorn et al., J. Biol.. Chem. 266: 16092 (1991)).

The relevance of ceramide in cell physiology has also been demonstrated by studies that examine the specific activity of ceramide analogues. For example, it has been shown that D-erythro-N-acetylsphingosine presents similar activities to natural ceramide (Bielawaska et al. J. Biol.. Chem. 268: 26226 (1993), Fishbein et al, J. Biol. Chem. 268:9255 (1993)), while its saturated analogue (D-erythro-N-acetyldihydrosphingosine), without the double bond in positions 4,5 lacks the effects of ceramide (Bielawaski et al., J. Biol. Chem. 268:26226 (1993), Tepper et al. Proc. Natl. Acad. Sci. U.S.A. 92: 8443 (1995)) although both compounds are incorporated and metabolized in an almost identical manner (Bielawska et al. J.Biol. Chem. 268: 26226 (1993)), that suggests that the lack of activity of the saturated derivative is due to its inability to interact with relevant targets. Indeed, D-erythro-N-acetylsphingosine, but not D-erythro-N-acetyldihydrosphingosine, activates the protein phosphatase CAPP in vitro (Fishbein et al. J. Biol. Chem. 268: 9255 (1993), Dobrowsky et al., J. Biol. Chem. 268: 15523 (1993)).

On the other hand, some of the effects produced by the ceramide have been achieved by manipulating its own metabolism. For example, by adding bacterial sphingomyelinase, that catalyses the hydrolysis of sphingomyelin to ceramide, an accumulation of ceramide occurs and the same effects are achieved as with addition of permeable ceramides (Okazaki et al, J. Biol. Chem. 264:19076 (1989), Mathias et al., Science 259: 519 (1993)). In another example, the PDMP and related compounds which inhibit ceramide glycosidation (Abe et al., J. Biochem. (Tokyo), 111:191(1992)) also lead to rises in intracellular levels of this lipid, producing a series of similar effects to those achieved with ceramide analogues.

These overall results strengthen the crucial role of ceramide in the regulation of different aspects of cell biology.

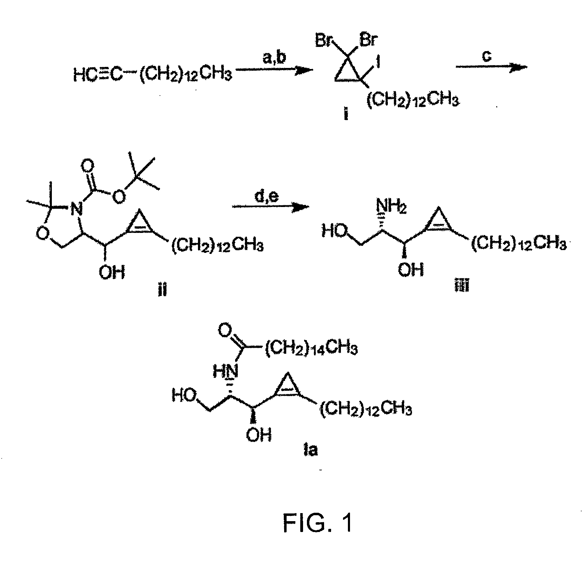

Ceramide is the central molecule in sphingolipid and glycosphingolipid biosynthesis (Figure 1). Ceramide is produced intracellularly via two metabolic pathways: the anabolic and the catabolic pathways. In the latter, ceramide is produced by hydrolysis of glycosphingolipids catalysed by hydrolases or hydrolysis of sphingomyelin ("sphingomyelin cycle"). The hydrolysis of sphingomyelin is mediated by sphingomyelinases (acidic or neutral), which are activated by a series of natural ligands (TNFa, interleukins etc.) and also by signals of cellular stress, such as ionizing radiations, pharmacological agents etc. On the other hand, the hydrolysis of sphingosine-1-phosphate by a phosphatase, followed by acylation of the amino group also leads to the production of a ceramide (salvage pathway).

The *de novo* biosynthesis of ceramide (anabolic pathway) is initiated by condensation of serine with palmitoyl CoA to form 3-ketodihydrosphingosine, which is then reduced to dihydrosphingosine. The acylation of dihydrosphingosine generates dihydroceramide, which, by the action of a desaturase, is transformed into ceramide. Ceramide then serves as a precursor of more complex lipids, such as sphingomyelin and glycosphingolipids (cerebrosides, gangliosides etc.) (Hannun, J. Biol. Chem. 269: 3125 (1994), Wiegandt in glycolipids (Weigandt, ed) pp. 199-259 , Elsevier, New York (1985), Merrill, Jr. and Jones Biochim. Biophys. Acta 1044:1 (1990), Van Echten and Sandhoff J. Biol. Chem. 268:53412 (1993), Hakomori, Annu. Rev. Biochem. 50: 733 (1981)). The transformation of ceramide into sphingomyelin involves the transfer of choline phosphate from a phosphatidylcholine molecule to the C1-OH of ceramide, generating diacylglycerol, which is another very important cell signalling mediator. Finally, ceramide can be transformed by hydrolysis of the amide function by the action of ceramidases into sphingosine, (Hannun, J. Biol. Chem. 269:3125 (1994), Spence et al. Biochem. Cell. Biol. 64:400 (19867), Slife et al., J. Biol. Chem. 264: 10371 (1989)) which, in turn, is phosphorylated at C1-OH by a kinase, giving rise to sphingosine-1-phosphate, which is also a very important lipidic mediator. By the action of sphingosine-1-phosphate lyase, the sphingosine-1-phosphate can be transformed into ethanolamine phosphate and 2-hexadecanal.

There are several clinical conditions that are caused by or associated with an accumulation of ceramide and the subsequent induction of apoptosis. Therefore, the development of molecules capable of blocking the enzymes involved in the production of ceramide constitutes a novel approach to discovering new pharmacological agents. In this context, different inhibitors have been described, both in scientific publications and in patents, for several of the enzymes involved in ceramide biosynthesis and metabolization, and have been the focus of a recent review [Kolter and Sandhoff, Angew. Chem. Int. Ed. 1999, 38:1532]. However, no inhibitor of dihydroceramide desaturase has been described as yet in the literature.

### DETAILED DESCRIPTION OF THE INVENTION

To date, no inhibitor of dihydroceramide desaturase has been reported in the literature. Since dihydroceramide desaturase is the last enzyme in the *de novo* biosynthesis of ceramide, one could expect type **I** compounds 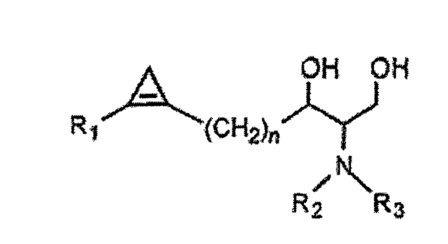 to be useful in the treatment of conditions derived from an accumulation of intracellular ceramide by increasing the *de novo* biosynthesis of ceramide. One of these conditions is diabetes associated with obesity in which an accumulation of saturated fatty acids leads to an increase in the *de novo* synthesis of ceramide in the pancreatic beta cells with the subsequent induction of apoptosis and destruction of these beta cells. The world patent WO 9944598 describes the use of fumonisine, an inhibitor of the N-acyltransferase of the dihydrosphingosine and, therefore, an inhibitor of *de novo* ceramide biosynthesis, as a method to prevent destruction of beta pancreatic cells and for the treatment of obesity-associated diabetes. Other clinical conditions derived from increased apoptosis induced by an accumulation of ceramide can also be treated with type I compounds. The present invention has arisen from the knowledge that specific cyclopropenic fatty acids of a certain structure potently and selectively inhibit the fatty acid desaturases (Salaun J; Baird, M.S. *Curr. Med. Chem.* **1995**, 2, 511-452, Johnson, A.R.; Pearson, J.A.; Shenstone, F.S.; Fogerty, A.C. Nature 1967, 214, 1244-1245, Johnson, A.R.; Fogerty A.C., Pearson, J.A.; Shenstone, F.S.; Bersten, A.M. *Lipids* **1968**, 4, 265-269, Fogerty, A.C.; Johnson, A.R.; Pearson, J.A. Lipids **1972**, 7, 335-338, Clark J.R. ; Kircher, H. W. *Lipids* **1972**, 7, 769-772, Raju, P. K.; Reiser, R. *J. Biol. Chem*. **1967**, 242, 379-384, Allen, E.; Johnson, A.R.; Fogerty, A.C.; Pearson, J. A. ; Shenstone, F.S. Lipids **1977**, 2, 419-423, Jeffcoat, R.; Pollard, M.R. Lipids **1977**, 12, 480-485, Ory, R.L.; Altschul, A.M. *Biochem. Biophys.. Res.* Commun. **1964**, 17, 12-16, Pande, S.V.; Mead, J.F.J. *Biol.* Chem **1970**, 245, 1856-1861, Arsequell, G.; Fabrias, G. Camps, F. Insect Biochem. **1989**, 19, 623-627, Gosalbo, L; Fabrias, G.; Arsequell, G.; Camps, F. Insect *Biochem.* Molec. Biol.**1992**, 22, 687-690, Gosalbo, L; Fabrias, G.; Camps, F. Arch. Insect *Biochem Physiol.* **1994**, 26, 279-286, Fabrias G.; Gosalbo, L; Quintana, J.; Camps, F.J. Lipid Res. **1996**, 37, 1503-1509, Fabrias, G.; Barrot, M.; Camps, F. Insect *Biochem Molec. Biol..* **1995**, 25, 655-660, Ando, T.; Ikemoto, K, Ohno, R.; Yamamoto, M. Arch. Insect Biochem. Physiol. **1998**, 37, 8-16, Ando, T.; Ohno, R.; Ikemoto, K.; Yamamoto, M. J. Agric. Food Chem. **1996**, 44, 3350-3354). Therefore, by analogy, type **I** compounds would be expected to be inhibitors of dihydroceramide desaturase.

Without limiting in any way the application of the present invention that is described in the claims specified in the following section, some aspects of this invention are described in detail in the following examples.

### EXPLANATION OF THE FIGURES

**Figure 1**. Synthesis of type **I** compounds. The steps of the synthesis are: a/HBr gas, tetraethylammonium bromide, b/tribromomethane, cetyltribenzylammonium bromide, 50% aqueous solution of sodium hydroxide; c/1, n-Butyl-lithium in tetrahydrofurane, 2, Garner aldehyde in tetrahydrofurane; 3, Column chromatography; d/ 1, trimethylsilyl triflate, 2,6-lutidine in tetrahydrofurane, 2, octanoyl chloride/pyridine in methanol/chloroform.

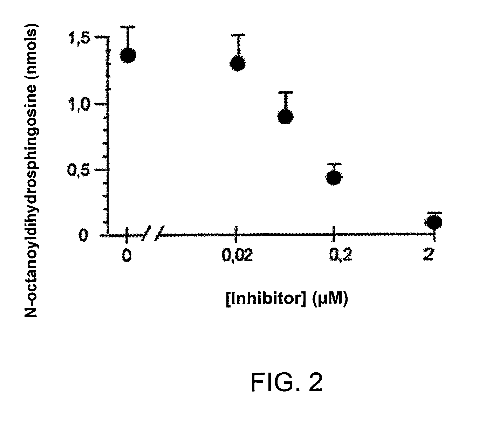

**Figure 2**. The effect of compound **la**, where R1 is C₁₃H₂₇, R₂ is C₈H₁₇ and R₃ is H on the dihydroceramide desaturase. The experiments involved incubation of microsomes with the la inhibitor at the doses indicated, the enzyme substrate (N-octanoyldihydrosphingosine) and NADH. The amounts of desaturated product formed are calculated on the basis of an internal standard (N-hexanoylsphingosine).

### EXAMPLES

### Example 1:

### Synthesis of la

The **la** compound is synthesized by condensation of tent-butyl (S)-(-)-4-formyl-2,2-dimethyl-3-oxazolidincarboxylate (Garner aldehyde) with 2-tridecyl-1-cyclopropenyl-lithium, obtained from 1-pentadecyne following the sequence of reactions described in Al Dulayami, J. R.; Baird, M. S.; Simpson, M.J.; Nyman, S. *Tetrahedron **1996**, 52,* 12509-12520. Two equivalents of n-butyl-lithium are added (1.5 M solution in hexane) to 1 equivalent of tribromocyclopropane (**i**) dissolved in anhydrous tetrahydrofurane maintaining the temperature below -50°C and then letting it rise gradually to 0°C. To the resulting solution, 1 equivalent of Garner aldehyde was added maintaining the temperature below -65°C. After stirring constantly for two hours a saturated solution of ammonium chloride was added and the extraction was carried out with diethyl ether. The evaporation of ether produces a mixture of diastereomeric alcohols erythro- and threo-**ii** (7:3), that are carefully separated by silica gel column chromatography eluting with a mixture of ethyl acetate/hexane at a ratio of 10:1. Each of the diastereomers is deprotected with trimethylsilyl triflate in accordance with the procedure described by Sakaitani, M.; Ohfune, Y. -. *Org. Chem.* **1990**, 55, 870-876, thus obtaining the aminodiols **iii**. The final product is obtained by acylation of the amine with octanoyl chloride (figure 2).
Spectroscopic data of **la**.
IR (NaCI): 1550, 1644, 3010, 3287 cm⁻¹
¹³C NMR (300 MHz, CDCl₃): 8.37 (CH₂), 14.04 (CH₃), 14.10 (CH₃), 22.59 (CH₂), 22.67 (CH₂), 25.71 (CH₂), 25.84 (CH₂), 27.29 (CH₂), 29.99 (CH₂), 29.20 (CH₂), 29.34 (CH₂), 29.44 (CH₂), 29.57 (CH₂), 29.64 (CH₂), 31.66 (CH₂), 31.90 (CH₂), 36.7 (CH₂), 53.70 (CHNH), 63.21 (CH₂OH), 70.66 (CHOH), 107.53 (CH), 115.50 (CH), 173.98 (NHCOR).
¹H NMR (200 MHz, CDCl₃): 0.87 (t, 6H, *J*=6.2), 1.01 (s, 1H), 1.25 (s, 28H), 1.60 (m. 4H), 2.24 (t, 2H, *J*= 7.2), 2.45 (dt, 2H, *J*=7.6, *J'*=1.2), 3.71 (dd, 1 H, *J* = 11.4, *J'*=3.4), 3.87 (dd, 1 H, *J*=11.2, *J'*=4), 4.18(m, 1H), 6.37 (d, 1 H NH).

### Example 2:

### Evaluation of the inhibitory activity of I on the dihydroceramide desaturase.

The inhibitory activity of the type **I** compounds that is claimed in this patent was tested on rat liver microsomes, prepared as described in [Michel, C. van Echten-Deckert, G.; Rother, J.; Sandhoff, K.; Wang, E.; Merrill, A. H. *J*. *Biol.* Chem. 1997, 272, 22432-22437]. The dihydroceramide desaturase activity was determined according to the procedure described in Michel, C. van Echten-Deckert, G.; Rother, J.; Sandhoff, K.; Wang, E.; Merrill, A. H. *J. Biol. Chem.* **1997**, 272, 22432-22437, with the following modifications: compound **la** in increasing doses of 0, 0.6, 1.25, 2.5, 5 and 10 nmoles, was solubilized in BSA-ethanol 9:1 (100µl) and to the resulting solution 10 nmoles of substrate dissolved in 10µl of ethanol were added. To this solution was added a total of 0.3 mg of the microsomal suspension containing the desaturase, and 30 µl of a 1µM solution of NADH in 0.1M phosphate buffer, pH 7.5. The suspension was incubated at 37°C for 30 minutes and the reactions were stopped by adding 0.5 mL of chloroform containing 1 nmol of N-hexanoylsphingosine as an internal standard for quantification. Repeated lipid extractions with chloroform were carried out (2 x 250 µl) and the combined organic extracts were evaporated to dryness under a stream of Nitrogen. The resulting residue was treated with 50 µl of bis-trimethylsilyltrifluoroacetamide at 25°C for 60 minutes. After this time, 50 µl of chloroform was added and the samples were kept at -80°C until their analysis. The analyses consisted of gas chromatography coupled to electron impact mass spectrometry (70 eV), using a Fisons series 8000 chromatograph and a Fisons MD-800 selective mass detector. The system was equipped with a Hewlett Packard HP-1 (30 m x 0.2 mm) capillary column.

Analysis of the lipidic extracts revealed that the effect of **la** on the dihydroceramide desaturase was dose-dependent with an IC_{50,} without preincubation of 0.052 µM (Figure 2).

## Claims

1. Derivatives of cyclopropenylceramide as desaturase inhibitors **characterised by** the general formula **I** in which R1 can be an alkyl, alkenyl, alkynyl or aryl or any heterocyclic group, n can have any value and can be branched or straight, with or without substitutions, and contain unsaturated carbons on the chain and R₂ and R₃ that can have the same or different values, represent aryl, heteroaryl, alkyl or acyl groups with one or more unsaturations in the chain that can also be branched or straight and can have or not have substitutions.

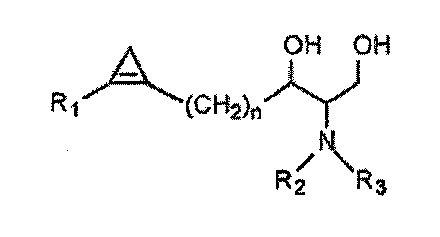

2. Use of derivatives of cyclopropenylceramide according to claim 1 as inhibitors of dihydroceramide desaturase.
